# EUROPÄISCHE PATENTSCHRIFT

(11) **EP 1 150 947 B1**
(45) Veröffentlichungstag und Bekanntmachung des Hinweises auf die Patenterteilung: **18.08.2004**
(21) Anmeldenummer: 00905013.9
(22) Anmeldetag: 03.02.2000
(51) Int. Cl.: C07C 301/00, C07C 303/22, C07C 303/26, C07C 309/04, C07C 309/66, C07D 327/10

(54) **VERFAHREN ZUR HERSTELLUNG VON ALKANSULFONSÄUREN**
METHOD FOR PRODUCING ALKANESULFONIC ACIDS
PROCEDE POUR PREPARER DES ACIDES SULFONIQUES D'ALCANE

(30) Priorität: 03.02.1999 DE 19904262
(43) Veröffentlichungstag der Anmeldung: 07.11.2001
(73) Patentinhaber: BASF AKTIENGESELLSCHAFT, 67056 Ludwigshafen (DE)
(72) Erfinder: HALBRITTER, Klaus, D-69124 Heidelberg (DE); STERZEL, Hans-Josef, D-67125 Dannstadt-Schauernheim (DE); EIERMANN, Matthias, D-67117 Limburgerhof (DE); FREUDENTHALER, Eva, D-67063 Ludwigshafen (DE)
(74) Vertreter: Isenbruck, Günter, Dr.
(86) Internationale Anmeldenummer: PCT/EP2000/000860
(87) Internationale Veröffentlichungsnummer: WO 2000/046188

(56) Entgegenhaltungen:
- DE-C- 925 473
- W.E. BISSINGER, ET AL.: "Rearrangement of alkyl sulphites to alkanesulphonate esters" JOURNAL OF THE AMERICAN CHEMICAL SOCIETY, Bd. 70, Nr. 11, 8. Dezember 1948 (1948-12-08), Seiten 3940-3941, XP002139818 American Chemical Society, Washington, DC, US ISSN: 0002-7863 in der Anmeldung erwähnt
- A. SIMON, ET AL.: "Über die Methylierung des Sulfitions und über die Umlagerungsmechanismen der Schwefelsäure- in Sulfonsäure-Abkömmlinge" CHEMISCHE BERICHTE, Bd. 89, Nr. 4, 1956, Seiten 883-894, XP002139819 Verlag Chemie, Weinheim, DE ISSN: 0009-2940 in der Anmeldung erwähnt
- A.J.W. BROOK, ET AL.: "The mechanism of the isomerisation of dimethyl sulphite to methyl methanesulphonate" JOURNAL OF THE CHEMICAL SOCIETY, SECTION B: PHYSICAL ORGANIC CHEMISTRY., Nr. 6, 1971, Seiten 1161-1163, XP002139820 Royal Society of Chemsitry, Letchworth, GB in der Anmeldung erwähnt
- R.E. DAVIS: "Hydrolysis of ethylene and dimethyl sulphite and the origin of strain in cyclic esters" JOURNAL OF THE AMERICAN CHEMICAL SOCIETY, Bd. 84, Nr. 4, 27. Februar 1962 (1962-02-27), Seiten 599-604, XP002139821 American Chemical Society, Washington, DC, US ISSN: 0002-7863 in der Anmeldung erwähnt
- A.V. DEVEKKI, ET AL.: "Coordination and catalytic activity of unsaturated compounds. IV. Interpretation of the mechanism of epoxidation of ethylene by sulphur dioxide" JOURNAL OF ORGANIC CHEMISTRY OF THE USSR. (ZHURNAL ORGANICHESKOI KHIMII), Bd. 19, Nr. 5, Mai 1983 (1983-05), Seiten 829-834, XP000914018 Consultants Bureau, New York, US ISSN: 0022-3271

## Beschreibung

Die Erfindung betrifft ein Verfahren zur Herstellung von Alkansulfonsäuren.

Alkansulfonsäuren werden in einer Reihe technischer Anwendungen eingesetzt. Langkettige Alkansulfonsäuren weisen z.B. Tensideigenschaften auf, während kurzkettige, wie Methansulfonsäure, z.B. als Hilfschemikalien zur galvanischen Abscheidung unedler Metalle wie Zinn oder Blei in der Verzinnung von Leiterplatten für die Elektronik oder in der Herstellung von Weißblech eingesetzt werden können.

In der Literatur sind eine Reihe von Verfahren zur Herstellung von Alkansulfonsäuren beschrieben. Dabei dienen insbesondere bei niederen Alkansulfonsäuren Alkylmercaptane oder Dialkyldisulfide als Ausgangsmaterialien, die üblicherweise durch Umsetzung von Schwefelwasserstoff mit Alkoholen hergestellt werden. Die Oxidationsreaktion der Alkylmercaptane oder der Dialkyldisulfide zu den entsprechenden Alkansulfonsäuren kann durch verschiedene Oxidationsmittel erreicht werden. So können als Oxidationsmittel Wasserstoffperoxid, Chlor, Dimethylsulfoxid und Iodwasserstoffsäure sowie die elektrochemische Oxidation eingesetzt werden.

Ein weiterer Weg zur Herstellung der Alkansulfonsäuren ist die Oxidation von Alkylmercaptanen oder Dialkyldisulfiden mit Sauerstoff in Gegenwart von Stickoxiden bzw. Salpetersäure. Die Oxidation mit Sauerstoff in Gegenwart von Salpetersäure wird z.B. in US 2,697,722 und US 2,727,920 beschrieben.

Diese Verfahren haben den Nachteil, daß sie von den äußerst giftigen bzw. unangenehm riechenden Substanzen Schwefelwasserstoff, Alkylmercaptan und/oder Dialkyldisulfid ausgehen. Diese werden bereits in geringsten Konzentrationen, die noch weit unterhalb der MAK (maximale Arbeitsplatzkonzentration)-Werte liegen, als äußerst geruchsbelästigend wahrgenommen und ihre Handhabung erfordert einen erheblichen sicherbeitstechnischen Aufwand.

Eine Aufgabe der vorliegenden Erfindung ist es daher, ein Verfahren zur Herstellung von Alkansulfonsäuren bereitzustellen, das den Einsatz dieser oben genannten giftigen und unangenehm riechenden Substanzen vermeidet und somit mit weniger sicherheitstechnischem Aufwand handhabar ist

Diese Aufgabe wird durch ein Verfahren zur Herstellung von Alkansulfonsäuren gelöst, umfassend die folgenden Schritte:
(a) Umsetzung eines Epoxids mit Schwefeldioxid, gegebenenfalls in Gegenwart eines Katalyastors, zu Alkylensulfit,
(b) Umsetzung des Alkylensulfits mit einem Alkohol, gegebenenfalls in Gegenwart eines Katalysators, zu Dialkylsulfit und dem entsprechenden Diol,
(c) Umlagerung des Dialkylsulfits in Gegenwart eines Katalysators zu dem entsprechenden Alkansulfonsäurealkylester,
(d) Hydrolyse des Alkansulfonsäurealkylesters zur gewünschten Alkansulfonsäure,
wobei einige der in den verschiedenen Schritten entstehenden Nebenprodukte in die entsprechenden Vorstufen zurückgeführt werden können, bzw. in Schritt (b) entstehendes Diol als Wertprodukt genutzt werden kann, und Schritt (b) kontinuierlich in einer Kolonne als Gegenstromverfahren durchgeführt wird.

### Umlagerung von Dialkylsirtfit zum entsprechenden Alkansulfonsäurealkylester

Die Umlagerung des Dialkylsulfits zum Alkansulfonsäurealkylester erfolgt in Gegenwart eines geeigneten Katalysators. Als Katalysatoren sind z.B. Basen wie Amine, bevorzugt tertiäre Amine, z.B. Dimethylanilin oder Tri-n-butylamin, oder heterocyclische Amine wie Piperidin oder Pyridin geeignet. Des weiteren sind Alkyliodid sowie quartäre Ammonium-, quartäre Phosphonium- oder tertiäre Sulfoniumsalze sowie Alkylierungsmittel geeignet Bevorzugt wird Tri-n-butylamin als Katalysator eingesetzt

Der Katalysator wird im allgemeinen in einer Menge von 0,01 bis 20 Mol-%, bevorzugt von 0,1 bis 10 Mol-%, besonders bevorzugt von 1 bis 5 Mol-% eingesetzt.

Üblicherweise wird die Umlagerung bei Temperaturen von 20 bis 250 °C, bevorzugt von 50 bis 200 °C, besonders bevorzugt von 120 bis 180°C durchgeführt.

Solche Umlagerungen sind in W. Voss et al., Justus Liebigs Ann. Chem. 485 (1931) 258-283; W. E. Bissinger et al., J. Am. Chem. Soc. 70 (1948) 3940; A. Simon et al., Chem. Ber. 89 (1956) 883; A. J. W. Brook et al., J. Chem. Soc. B (1971) 1061, beschrieben.

Die nach Reaktionsende erhaltene, den Alkansulfonsäurealkylester enthaltende Reaktionsmischung wird gegebenenfalls aufgearbeitet, bevorzugt durch Destillation, besonders bevorzugt durch Vakuumdestillation, um zu hohe Sumpftemperaturen und somit eine häufig stattfindende teilweise Zersetzung des Reaktionsproduktes zu vermeiden.

Eine Destillation der den Alkansulfonsäurealkylester enthaltenden Reaktionsmischung ist insofern vorteilhaft, da eine Destillation der im nachfolgenden Schritt hergestellten Alkansulfonsäure auf diese Weise gegebenenfalls vermieden werden kann. Eine Säuredestillation, wie die der Alkansulfonsäure, muß im allgemeinen in korrosionsbeständigen Apparaturen bei hohen Temperaturen durchgeführt werden, wohingegen die Destillation des entsprechenden Esters im allgemeinen bei tieferen Temperaturen und in einer einfacheren Destillationskolonne aus nicht korrosionsbeständigem Material durchgeführt werden kann.

Das Reaktionsprodukt dieser Stufe, der Alkansulfonsäurealkylester, kann in Ausbeuten von im allgemeinen >60% (bezogen auf das eingesetzte Dialkylsulfit) erhalten werden.

### Hydrolyse des Alkansulfonsäurealkylesters zur Alkansulfonsäure

Die Hydrolyse des Alkansulfonsäurealkylesters zu der entsprechenden Alkansulfonsäure kann direkt mit Wasser erfolgen (W. Voss et al., Justus Liebigs Ann. Chem. 485 (1931) 265; P. M. Laughton et al., Can. J. Chem. 34 (1956) 1714 -1718).

Im allgemeinen wird die Hydrolyse bei erhöhter Temperatur ohne Katalysator durchgeführt. Dazu wird Wasser in einer Menge im allgemeinen von 50 bis 500 Mol-%, bevorzugt von 100 bis 200 Mol-%, besonders bevorzugt von 120 Mol-% zugegeben. Die Hydrolyse erfolgt bei Temperaturen im allgemeinen von 80 bis 180 °C, bevorzugt von 100 bis 150°C, besonders bevorzugt von 120°C. Die Hydrolyse erfolgt bevorzugt im kontinuierlich betriebenen Reaktor mit aufgesetzter Kolonne, über die der freigesetzte Alkohol abgetrennt wird.

Bei der Hydrolyse entsteht die gewünschte Alkansulfonsäure, üblicherweise in wäßriger Lösung, und der dem eingesetzten Alkansulfonsäurealkylester entsprechende Alkohol. Der Alkohol wird abdestilliert. Wird eine nicht-wäßrige Alkansulfonsäure benötigt, muß die wäßrige Lösung aufgearbeitet werden, z.B. durch Destillation.

### Synthese von Dialkylsulfit

Das zur Synthese des Alkansulfonsäurealkylesters eingesetzte Dialkylsulfit wird durch Umsetzung von Alkylensulfit mit einem Alkohol erhalten. Als Nebenprodukt entsteht dabei das dem Alkylensulfit entsprechende Diol. Als Alkohol wird üblicherweise ein einwertiger Alkohol, also ein Alkohol mit einer OH-Gruppe, eingesetzt. Der Alkylrest des eingesetzten Alkohols entspricht dem der als Endprodukt gewünschten Alkansulfonsäure.

### Als Alkylensulfit können Alkylensulfite der allgemeinen Formel I eingesetzt werden:

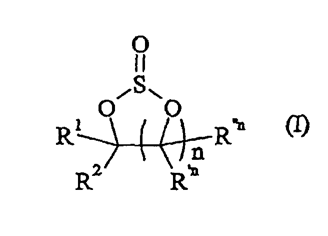

In der Formel I ist n im allgemeinen 1 bis 6, bevorzugt 1 bis 4, besonders bevorzugt 1 bis 2, ganz besonders bevorzugt 1. R¹, R², R^{'n} und R^{"n} sind unabhängig voneinander Wasserstoff, Alkyl- oder Arylreste. Besonders bevorzugt sind R¹, R², R^{'n} und R^{"n} unabhängig voneinander Wasserstoff oder Methylreste. Ganz besonders bevorzugt werden Ethylensulfit und Propylensulfit eingesetzt.

Das bei dieser Umsetzung als Nebenprodukt entstehende Diol, ganz besonders bevorzugt Ethylenglycol, findet in der Technik vielseitige Verwendung, beispielsweise als Komponente für Kühlflüssigkeiten, Medium zur Wärmeübertragung, hydraulische Flüssigkeit, Lösungsmittel oder Ausgangsmaterial für weitere Sythesen.

Diese Umsetzung des Alkylensulfits mit einem Alkohol zu dem gewünschten Dialkylsulfit und dem dem Alkylensulfit entsprechenden Diol ist eine Gleichgewichtsreaktion. Um eine Gleichgewichtsverschiebung in Richtung des Dialkylsulfits zu erreichen, ist eine fortwährende Entfernung des gebildeten Diols erforderlich.

Eine einfache fortwährende Destillation des Diols aus dem Reaktionsgemisch ist im allgemeinen nicht möglich, da, insbesondere bei der Herstellung von Dimethylsulfit, üblicherweise der eingesetzte Alkohol die am niedrigsten siedende Komponente darstellt und daher zuerst abdestillieren würde. Das würde eine Gleichgewichtsverschiebung nach links, also in Richtung der Ausgangsstoffe, zur Folge haben.

Eine im allgemeinen quantitative Umsetzung des Alkylensulfits mit einem Alkohol zum gewünschten Dialkylsulfit ist jedoch in einer Kolonne nach einem in der nicht vorveröffentlichten, gleichzeitig eingereichten Anmeldung DE-A 199 04 261 mit dem Titel "Verfahren zur Herstellung von Dimethylsulfit" beschriebenen Verfahren möglich.

Bei diesem Verfahren handelt es sich um ein Verfahren zur Herstellung von Dimethylsulfit durch Umsetzung (Umesterung) eines cyclischen Alkylensulfits mit mindestens 2 Kohlenstoffatomen mit Methanol, gegebenenfalls in Gegenwart eines Katalysators, wobei das Verfahren kontinuierlich in einer Kolonne durchgeführt wird.

Das Verfahren wird im Gegenstromverfahren durchgeführt, wobei Methanol im unteren Teil der Kolonne zugegeben wird und im oberen Teil der Kolonne zugegebenem cyclischem Alkylensulfit entgegenströmt Das bei der Umsetzung (Umesterung) dem eingesetzten cyclischen Alkylensulfit entsprechende freigesetzte Diol wird über den Sumpf der Kolonne und das entstehende Dimethylsulfit, zusammen mit nicht umgesetztem Methanol, über den Kopf der Kolonne kontinuierlich abgezogen. Auf diese Weise wird eine im allgemeinen vollständige Umsetzung des eingesetzten Alkylensulfits erreicht.

Die Umsetzung von Alkylensulfit mit einem Alkohol zu Dialkylsulfit und Diol kann gegebenenfalls in Gegenwart eines Katalysators erfolgen. Als Katalysatoren sind im allgemeinen saure oder basische Substanzen geeignet. Beispiele für geeignete Katalysatoren sind in R. E. Davis, J. Am. Chem. Soc. 84 (1992) 599 - 604; P. A. Bristow et al., Tetrahedron Letters 10 (1967) 901 - 903; P. A. Bristow et al., J. Chem. Soc. C (1968) 685 - 687 beschrieben. Bevorzugt wird Methansulfonsäure als Katalysator eingesetzt. Üblicherweise wird der Katalysator in einer Menge von 0,01 bis 5 Mol-%, bevorzugt von 0,1 bis 1 Mol-% eingesetzt

### Synthese des Alkylensulfits

Die Synthese des zur Herstellung des Dialkylsulfits eingesetzten Alkylensulfits erfolgt durch Umsetzung von Epoxiden mit Schwefeldioxid, gegebenenfalls in Gegenwart eines Katalysators.

Als Epoxide können Epoxide der allgemeinen Formel II, entsprechend den gewünschten Alkylensulfiten, eingesetzt werden:

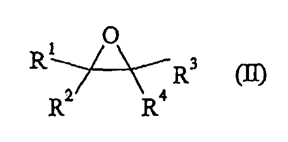

In der Formel II sind R¹, R², R³ und R⁴ unabhängig voneinander Wasserstoff, Alkyloder Arylreste, bevorzugt sind R¹, R², R³ und R⁴ unabhängig voneinander Wasserstoff oder Methylreste. Besonders bevorzugt werden Ethylenoxid und Propylenoxid eingesetzt. Ganz besonders bevorzugt wird Ethylenoxid eingesetzt

Gegebenenfalls können Katalysatoren wie Tetraethylammoniumhalogenide oder Amine zugesetzt werden. Die Verwendung solcher Katalysatoren ist in G. A. Razuvaev et al., 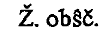 Chim. 31 (1961) 1328 - 1332 und GB-A 844 104 beschrieben.

Der Katalysator wird im allgemeinen in einer Menge von 0,01 bis 10 Gew-%, bevorzugt von 0,1 bis 5 Gew-%, besonders bevorzugt von 1 Gew-% zugesetzt.

Das in Schritt (c) als Nebenprodukt entstehende Schwefeldioxid kann in Schritt (a) zurückgeführt werden, und der in Schritt (c) entstehende Dialkylether kann nach Hydrolyse zum entsprechenden Alkohol in Schritt (b) zurückgeführt werden. Der in Schritt (d) entstehende Alkohol kann ebenfalls in Schritt (b) zurückgeführt werden.

Vorzugsweise wird der in Schritt (c) hergestellte Alkansulfonsäurealkylester vor der Hydrolyse zur entsprechenden Alkansulfonsäure (Schritt (d)) aufgereinigt.

Bevorzugt weisen die Alkansulfonsäuren Alkylreste mit 1 bis 3 Kohlenstoffatomen auf, wie Methyl-, Ethyl-, n-Propyl- oder i-Propylreste, wovon der Methylrest ganz besonders bevorzugt ist

Daraus ergibt sich, daß als Alkohol (Schritt (b)) besonders bevorzugt Alkohole mit 1 bis 3 Kohlenstoffatomen wie Methanol, Ethanol, n-Propanol oder i-Propanol, ganz besonders bevorzugt Methanol, eingesetzt werden.

Die besonders bevorzugt eingesetzten Dialkylsulfite (Schritt (c)) sind solche mit 1 bis 3 Kohlenstoffatomen in ihren Alkylresten wie Dimethylsulfit, Diethylsulfit, Di-npropylsulfit oder Di-i-propylsulfit, ganz besonders bevorzugt ist Dimethylsulfit.

Die eingesetzten Alkansulfonsäureslkylester (Schritt (d)) sind demgemäß solche mit besonders bevorzugt 1 bis 3 Kohlenstoffatomen, ganz besonders bevorzugt einem Kohlenstoffatom im Alkylrest. Bevorzugt sind der Alkylrest und der Rest in der Estergruppe dieselben, so daß Methansulfonsäuremethylester ganz besonders bevorzugt ist

Das nachfolgende Beispiel erläutert die Erfindung zusätzlich.

### Beispiel

### Synthese von Ethylensulfit ausgehend von Ethylenoxid und SO₂:

In einem Druckreaktor wurden 5 g Tetraethylammoniumbromid gelöst in 500 g Ethylensulfit vorgelegt und auf 170°C erhitzt. Anschließend wurden langsam 408 g Ethylenoxid und 622 g SO₂ aufgegast. Der Druck lag dabei zwischen 4 und 8,5 bar. Nach der Reaktion wurde ein Rohprodukt erhalten, welches zu 96% (GC-Flächenprozent) aus Ethylensulfit bestand.

### Synthese von Dimethylsulfit ausgehend von Ethylensulfit

Auf den 40sten Boden einer Glockenbodenkolonne (60 Böden, ⌀ 43 mm) wurde ein Strom von 100g/h Ethylensulfit und 0,44 g/h Methansulfonsäure flüssig zugefahren (Strom 1). Im Sumpf der Kolonne wurde temperaturgeregelt ein Strom von 400 g/h dampfförmigem Methanol zudosiert. Der Großteil des Methanols wurde aus der zweiten Kolonne zurückgeführt und temperaturgeregelt mit frischem Methanol (60 g/h) ergänzt (Strom 2). Der Sumpf der Kolonne wurde auf 192°C geheizt und standgeregelt abgepumpt (Strom 3). Er bestand überwiegend aus Ethylenglykol neben wenig Oligoethylenglykol und Katalysator. Die Brüden der Kolonne wurden mittels Rückflußkühler auf 75°C gekühlt und in eine zweite Kolonne (Packungskolonne, ∅ 43 mm, 2 m Laborgewebepackung, 1000 cm²/cm³ Oberfläche) gefahren. Diese wurde bei 800 mbar Kopfdruck betrieben. Der Sumpf der zweiten Kolonne wurde auf 105°C aufgeheizt. Er enthielt das Dimethylsulfit und wurde standgeregelt ausgetragen (Strom 4). Das Rücklaufverhältnis wurde so eingestellt, daß die Temperatur im oberen Teil der Kolonne 58°C betrug.

### Umlagerung von Dimethylsulfit zu Methansulfonsäuremethylester

In einem 500 ml Kolben mit aufgesetztem Rückflußkühler wurden 220 g Dimethylsulfit und 18,5 g Tri-n-butylamin vorgelegt und im Ölbad unter Rückfluß auf 130°C erhitzt. Die Temperatur wurde mit zunehmendem Umsatz über einen Zeitraum von 8 h auf 170°C erhöht. Dabei färbte sich die Mischung gegen Ende der Reaktion dunkel. Nach Aufarbeitung des Rohprodukts durch Vakuumdestillation (62°C, 6 mbar) wurden 143 g (Ausbeute: 65%) Methansulfonsäuremethylester erhalten.

### Gewinnung von Methansulfonsäure durch Hydrolyse von Methansulfonsäuremethylester

Eine Mischung aus 80 g Methansulfonsäuremethylester und 130,9 g H₂O wurde unter starkem Rühren auf 98°C erhitzt. Gemäß ¹H-NMR spektroskopischer Analyse war der Ester nach 45 min vollständig hydrolysiert. Das Reaktionsgemisch enthielt neben Wasser nur Methansulfonsäure und Methanol. Ein Teil des freigesetzten Methanols reagierte im Verlauf der Hydrolyse zu Dimethylether, welcher gasförmig abging.

## Patentansprüche

1. Verfahren zur Herstellung von Alkansulfonsäuren, umfassend die folgenden Schritte:
(a) Umsetzung von einem Epoxid mit Schwefeldioxid, gegebenenfalls in Gegenwart eines Katalysators, zu Alkylensulfit,
(b) Umsetzung des Alkylensulfits mit einem Alkohol, gegebenenfalls in Gegenwart eines Katalysators, zu Dialkylsulfit und dem entsprechenden Diol,
(c) Umlagerung des Dialkylsulfits in Gegenwart eines Katalysators zu dem entsprechenden Alkansulfonsäurealkylester,
(d) Hydrolyse des Alkansulfonsäurealkylesters zur gewünschten Alkansulfonsäure,
wobei die in den verschiedenen Schritten entstehenden Nebenprodukte in die entsprechenden Vorstufen zurückgeführt werden können, bzw. in Schritt (b) entstehendes Diol als Wertprodukt genutzt werden kann, und Schritt (b) kontinuierlich in einer Kolonne als Gegenstromverfahren durchgeführt wird.

2. Verfahren nach Anspruch 1, **dadurch gekennzeichnet, daß** der hergestellte Alkansulfonsäurealkylester vor der Hydrolyse zur entsprechendem Alkansulfonsäure aufgereinigt wird.

3. Verfahren nach Anspruche 1 oder 2, **dadurch gekennzeichnet, daß** die Alkansulfonsäuren Alkylreste mit 1 bis 3 Kohlenstoffatomen aufweisen.

4. Verfahren nach Anspruch 3, **dadurch gekennzeichnet, daß** die Alkylreste Methylreste sind.

## Claims

1. A process for preparing alkanesulfonic acids, which comprises the following steps:
(a) reaction of an epoxide with sulfur dioxide, in the presence or absence of a catalyst, to form alkylene sulfite,
(b) reaction of the alkylene sulfite with an alcohol, in the presence or absence of a catalyst, to form dialkyl sulfite and the corresponding diol,
(c) rearrangement of the dialkyl sulfite in the presence of a catalyst to form the corresponding alkyl alkanesulfonate,
(d) hydrolysis of the alkyl alkanesulfonate to give the desired alkanesulfonic acid,
where some of the by-products formed in the various steps can be recirculated to the appropriate preceding steps or the diol formed in step (b) can be utilized as useful product, and step (b) is carried out continuously in a column as a countercurrent process.

2. A process as claimed in claim 1, wherein the alkyl alkanesulfonate prepared is purified prior to hydrolysis to the corresponding alkanesulfonic acid.

3. A process as claimed in claim 1, wherein the alkanesulfonic acids have alkyl radicals having from 1 to 3 carbon atoms.

4. A process as claimed in claim 3, wherein the alkyl radical is a methyl radical.

## Revendications

1. Procédé pour la préparation d'acides alcanesulfoniques, comprenant les étapes suivantes:
(a) Réaction d'un époxyde avec du dioxyde de soufre, éventuellement en présence d'un catalyseur, pour former du sulfite d'alkylène,
(b) Réaction du sulfite d'alkylène avec un alcool, éventuellement en présence d'un catalyseur, pour former du sulfite de dialkyle et le diol correspondant,
(c) Réarrangement du sulfite de dialkyle en présence d'un catalyseur en l'alkylester d'acide alcanesulfonique correspondant,
(d) Hydrolyse de l'alkylester d'acide alcanesulfonique pour former l'acide alcanesulfonique souhaité,
où les sous-produits formés dans les différentes étapes peuvent être recyclés dans les phases préalables correspondantes, par exemple le diol formé à l'étape (b) peut être utilisé comme produit valorisable, et l'étape (b) est entreprise en continu dans une colonne sous la forme d'un procédé en contre-courant.

2. Procédé selon le revendication 1, **caractérisé en ce que** l'alkylester d'acide alcanesulfonique préparé est purifié avant l'hydrolyse en l'acide alcanesulfonique correspondant.

3. Procédé selon la revendication 1 ou 2, **caractérisé en ce que** les acides alcanesulfoniques présentent des radicaux alkyle comportant de 1 à 3 atomes de carbone.

4. Procédé selon la revendication 3, **caractérisé en ce que** les radicaux alkyle sont des radicaux méthyle.
